# EUROPEAN PATENT SPECIFICATION

(11) **EP 2 288 317 B1**
(45) Date of publication and mention of the grant of the patent: **25.09.2013**
(21) Application number: 09717225.8
(22) Date of filing: 06.03.2009
(51) Int. Cl.: A61F 5/05

(54) **PROTECTIVE COLLAR WITH FIXING MEANS TO THE USER'S BODY**
SCHUTZKRAGEN MIT MITTEL ZUR FIXIERUNG AM KÖRPER DES ANWENDERS
COLLIER PROTECTEUR AVEC MOYEN DE FIXATION AU CORPS D'UN UTILISATEUR

(30) Priority: 06.03.2008 IT TV20080039
(43) Date of publication of application: 02.03.2011
(73) Proprietor: Alpinestars Research S.R.L., 31010 Coste di Maser (Treviso) (IT)
(72) Inventor: MAZZAROLO, Giovanni, I-31010 Coste Di Maser (Treviso) (IT); BALLANTYNE, Colin, I-31011 Caselle D'asolo (Treviso) (IT)
(74) Representative: Dragotti, Gianfranco
(86) International application number: PCT/IB2009/050934
(87) International publication number: WO 2009/109941

(56) References cited:
- EP-A- 0 023 115
- WO-A-2005/051251
- US-A- 4 502 471
- US-A- 5 590 826

## Description

This invention relates to a protective collar, useful during sporting activities, especially when riding motorcycles.

Various protective collars intended for persons who practise sporting activities are known and are commercially available. The obvious objective is to protect a part of the human body, such as the neck, which is extremely delicate and prone to fractures and knocks. In the event of a fall, for example from a motorcycle travelling at speed, it has been known that the neck often suffers damage, despite the fact that the rider is wearing a conventional protective helmet.

An example of a collar for moto-cross is described in the international patent application No. WO 2005/051251 in the name of Leatt.

This collar consists of two sections which are releasably connected together by means of a side hinge. The collar has surfaces which are directed upwards and limit the inclination of the head in all directions when the helmet is worn.

The collar rests on the user's shoulders by means of a padding permanently fixed to its bottom edge and has a plate extending at the rear downwards from its rear edge, with the function of transferring to the sides of the spinal column loads acting on the user's back, so that they are unable to act on the spine itself.

This type of collar, as well as other collars which are known from the prior art, however, have various problems and drawbacks.

Firstly, often the riders require to use numerous additional buckles and straps with the collar in order to fix it firmly on the shoulders.

In the solution according to WO 2005/051251 the recessed shape of the collar is exploited for this purpose so as to insert between two edges, along the sides, two annular, elastic, fixing straps. The (not insignificant) disadvantage is that the straps must necessarily be worn on top of the riding suit or other external clothing, and this, in addition to being uncomfortable, is dangerous because of the real risk that the straps may get caught in projecting parts of the motorcycle.

The object of the invention is to provide a collar which solves substantially the abovementioned problems and drawbacks and is easy to use, in addition to protecting the user in an effective manner.

In particular, the object of the present invention is to provide a collar which can be easily fixed by the user to his body.

This object is achieved with a collar as defined in claim 1.

The advantages of the invention will be explained more fully by the following description, intended purely by way of a non-limiting example, of a preferred embodiment of a collar, shown in the accompanying drawings in which:
Figure 1 shows a three-dimensional front view of the collar;
Figure 2 shows a three-dimensional rear view of the collar according to Fig. 1;
Figure 3 shows fixing straps for the collar;

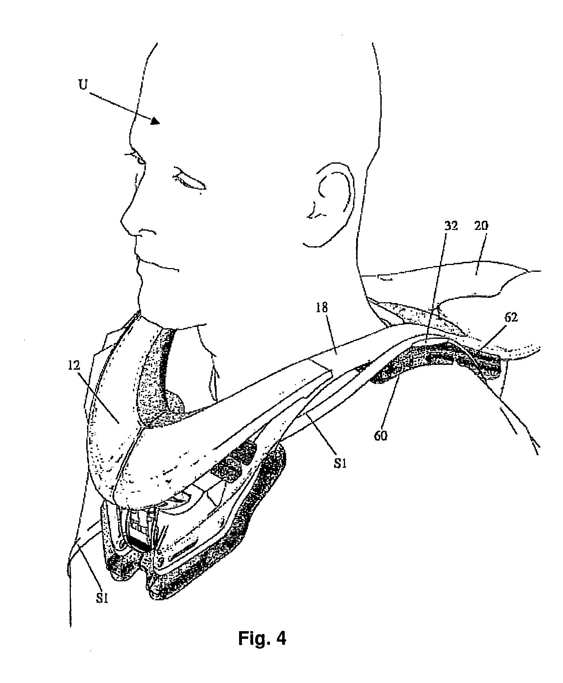

Figure 4 shows the straps connected to the collar according to Fig. 1;
Figure 5 shows a top plan view of Fig. 4;

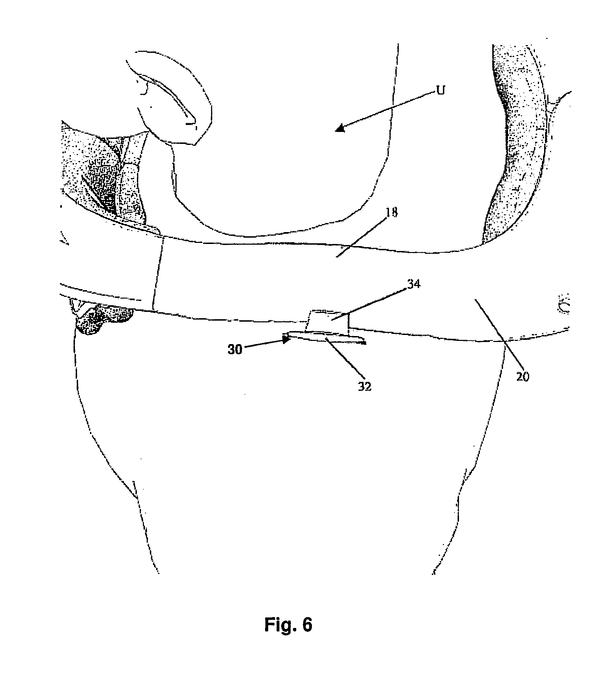

Figure 6 shows a top plan view of Fig. 4 without fixing strap.
Figure 7 shows a top plan view of Fig. 4 with the fixing strap connected to the collar in an alternative manner.

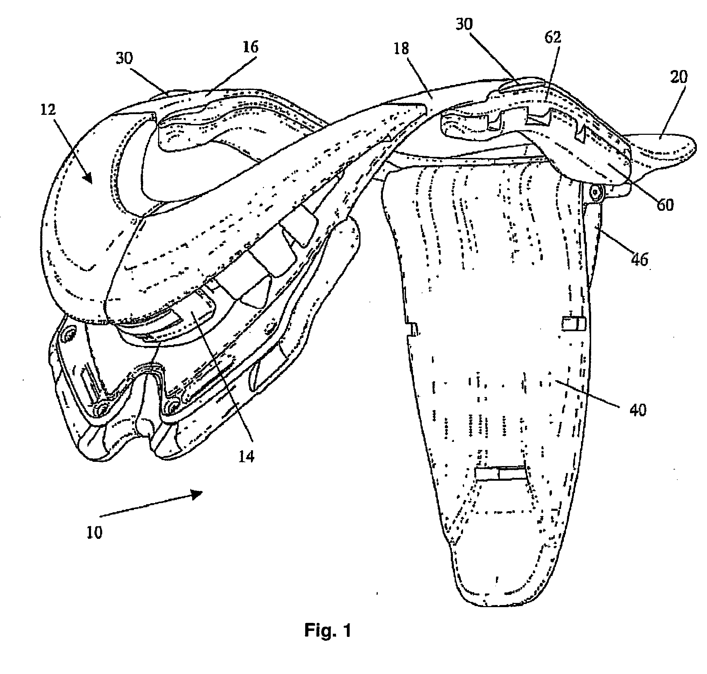

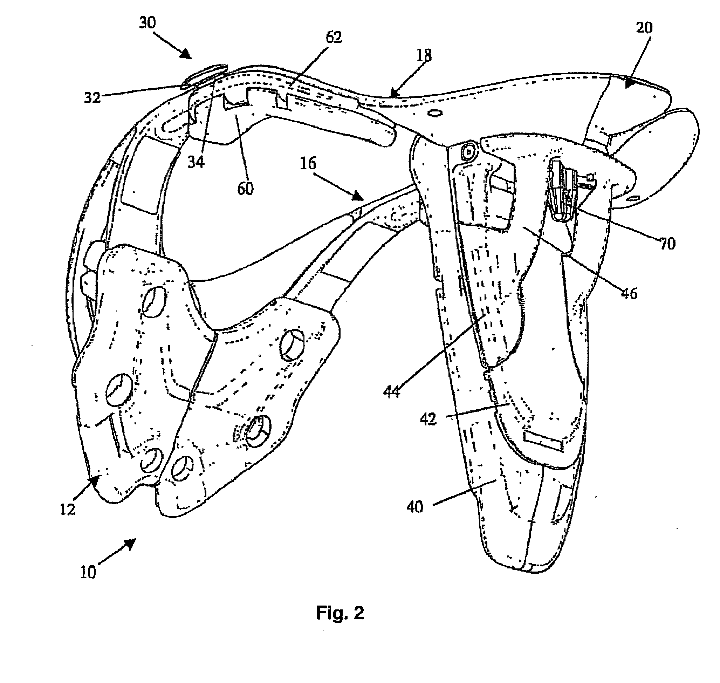

With reference first of all to Figures 1 and 2, there is shown a collar 10 which has a symmetrical structure and consists of a front part 12, which rests on the chest, closed by a fastening lever 14, two side segments 16, 18 which are nearly flat, and a rear part 20.

The segments 16, 18 are hinged together by means of a rear pin 70 and may be opened wide apart so that the neck can be inserted inside the collar 10. The fastening lever 14 has the function of fastening together the segments 16, 18 in the closed position such that, during use, the collar 10 has an annular structure which surrounds the neck completely.

The segments 16, 18 have a cross-section in the form of a rounded rectangle and each of them has, mounted on the edge towards the outside of the collar 10, a hooked tongue 30 which is formed by a central body 34 which terminates in a right-angled extension 32 widened in the form of a mushroom head.

Two pads 60, 62 are mounted underneath each of the segments 16, 18, being arranged on top of each other.

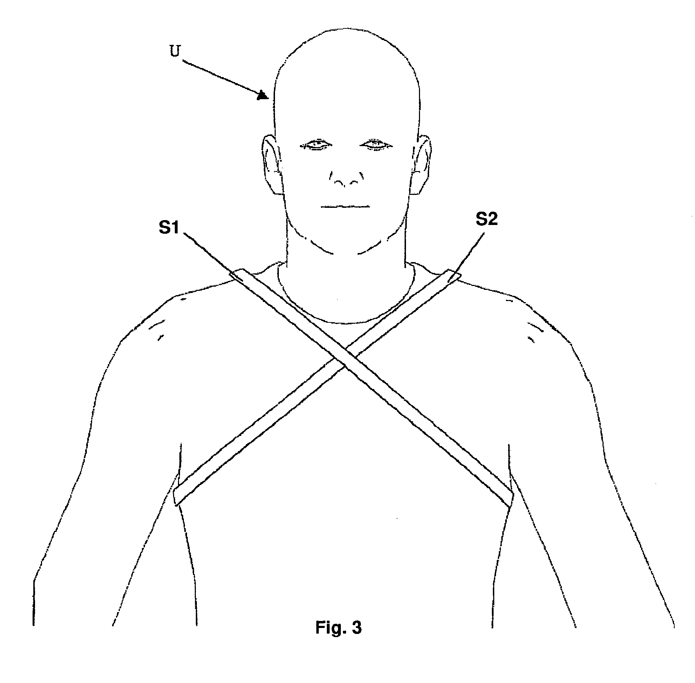

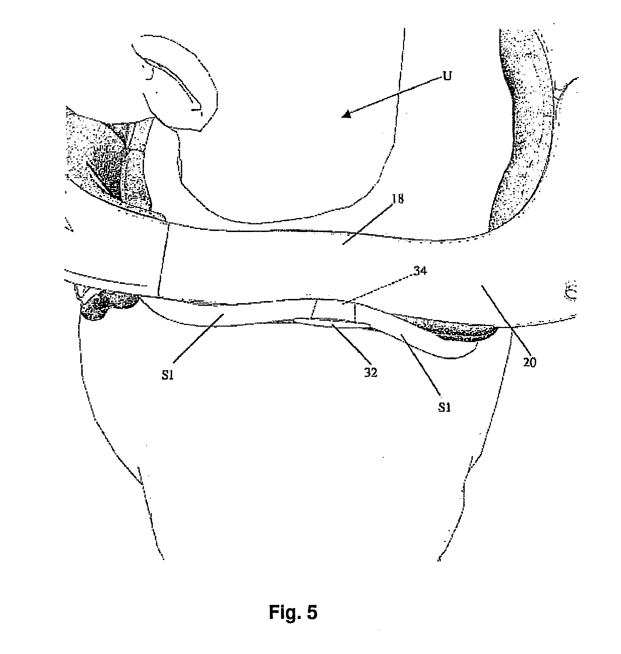

A user U (Fig. 3) who wishes to fix the collar 10 may use two elastic straps S1, S2 which are arranged so as to cross over each other on the chest. With the collar already positioned around the neck, it is then merely required to take hold of each strap and raise it so as to cause it to engage with the nearest tongue 30, passing over the extension or head 32 (Fig. 5). The tension of the straps S1, S2 ensures that the collar 10 remains tethered to the user's body such that small movements of the collar 10 are permitted, but the overall downward force of the straps S1, S2 always returns the collar 10 to its original position on the user's body.

The straps S1, S2 cannot come free of the tongue 30 because they are retained by the extension 32, unless a deliberate action is performed by the user U.

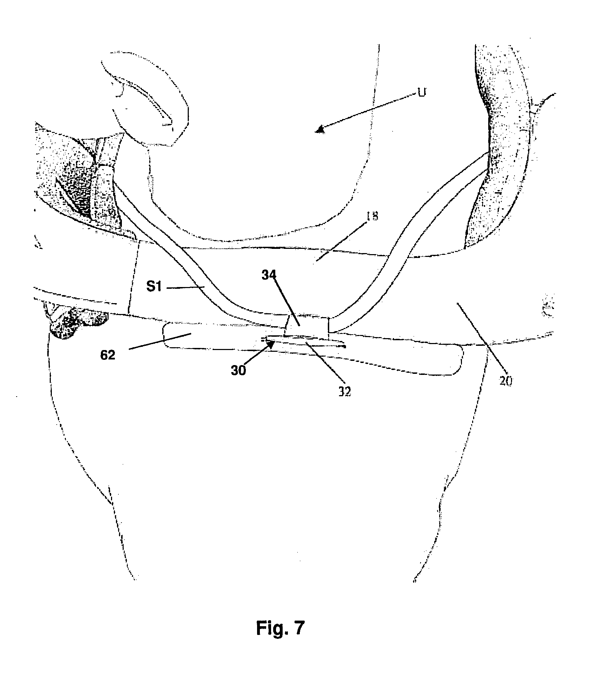

Thanks to the universal shape of the tongue 30 and the extension 32, figure 7, embodies an alternative fixing method of the straps S1, S2 whereby the straps S1, S2 are routed over the top surface 18 of the collar 10. The user U engages the straps S1, S2 with the tongue 30 by extending the straps S1, S2 over the top surface 18 and under the extension 32.

It should be noted that co-operation between the tongue 30 and straps S1, S2 also functions when the straps S1, S2 are used underneath clothing worn by the user U since the upper part of each strap may be extracted through the neck opening in the clothing. The embodiment in figure 7 also improves on this by requiring the collar of the clothing worn to be stretched open less.

According to a variant, the straps S1, S2 are joined permanently to the collar, for example precisely to a tongue such as that indicated by 30. In this case the straps S1, S2 do not necessarily forms a closed loop but may be open and have adjusting/closing means, such as a belt for tightening or Velcro® parts to be fixed together.

From the above description it is clear that the collar according to the present invention has characteristics such as to salve advantageously the problems and drawbacks of the devices according to the prior art.

## Claims

1. Collar (10) for protecting the neck of a user, useful in particular for motorcycle riders, comprising:
two half-collars (16,18) which are pivotably hinged together at one end so as to be rotatable in a substantially horizontal plane and which can be connected together by means of a fastening and release lever (14);
a plate (42) which at the rear extends downwards from the bottom edge of the collar so as to be centred with respect to the spinal column of the user,
a protective shield (12) which extends downwards at the front,
securing means (30) for temporarily or permanently connecting at least one strap (S1, S2) for fixing the collar (10) to the user's body,
**characterized in that**
said securing means comprise a tongue-like member (30) projecting laterally from the collar (10).

2. Protective collar (10) according to Claim 1 **characterized in that** said tongue-like member (30) is formed by a central body (34) which terminates in a right-angled extension (32).

3. Protective collar (10) according to Claim 1 or 2, **characterized in that** said tongue (30) has a widened mushroom-like head (32) for keeping the associated strap (S1, S2) in position.

4. Protective collar (10) according to any of preceding claims, **characterized in that** said tongue-like securing means (32) are two in number, one for each side (16, 18) of the collar (10).

5. Protective collar (10) according to any of preceding claims, further comprising at least one elastic strap (S1, S2) suitable for fixing the protective collar (10) to the body of the user said at least one elastic strap (S1, S2) being arranged so as to be crossed on the chest; said strap (S1, S2) being suitable to co-operate with said securing means (30), passing either over or under the extension (32), also when it is used underneath clothing.

6. Protective collar (10) according to any of preceding claims, comprising at least one strap (S1, S2) joined permanently to said securing means (30); said strap (S1, S2) being open and having adjusting/closing means.

7. Protective collar (10) according to Claim 6, wherein said adjusting/closing means comprise a belt for tightening or Velcro® parts to be fixed together.

## Patentansprüche

1. Kragen (10) zum Schützen des Nackens eines Benutzers, der insbesondere für Motorradfahrer nützlich ist, mit:
zwei Halbkragen (16, 18), die schwenkbar an einem Ende aneinander gelenkt sind, um so in einer im Wesentlichen horizontalen Ebene drehbar zu sein, und die mittels eines Befestigungs- und Lösehebels (14) miteinander verbunden werden können;
einer Platte (42), die sich an der hinteren Seite von der unteren Kante des Kragens nach unten erstreckt, um so hinsichtlich der Wirbelsäule des Benutzers zentriert zu sein,
einem Schutzschild (12), das sich an der vorderen Seite nach unten erstreckt,
einer Sicherungseinrichtung (30) zum vorübergehenden oder dauerhaften Verbinden zumindest eines Streifens (S1, S2) zum Befestigen des Kragens (10) an dem Körper des Benutzers,
**dadurch gekennzeichnet, dass** die Sicherungseinrichtung ein zungenartiges Element (30) aufweist, das von dem Kragen (10) seitlich vorsteht.

2. Schutzkragen (10) gemäß Anspruch 1, **dadurch gekennzeichnet, dass** das zungenartige Element (30) durch einen zentralen Körper (34) ausgebildet ist, der in einer rechtwinkligen Erweiterung (32) endet.

3. Schutzkragen (10) gemäß Anspruch 1 oder 2, **dadurch gekennzeichnet, dass** die Zunge (30) einen aufgeweiteten, pilzförmigen Kopf (32) hat, um dem dazugehörigen Streifen (S1, S2) in Position zu halten.

4. Schutzkragen (10) gemäß einem der vorherigen Ansprüche, **dadurch gekennzeichnet, dass** die zungenartige
Sicherungseinrichtung (32) zweifach vorhanden ist, und zwar eine für jede Seite (16, 18) des Kragens (10).

5. Schutzkragen (10) gemäß einem der vorherigen Ansprüche, des Weiteren mit zumindest einem elastischen Streifen (S1, S2), der zum Befestigen des Schutzkragens (10) an den Körper des Benutzers geeignet ist, wobei der zumindest eine elastische Streifen (S1, S2) so angeordnet ist, dass er die Brust kreuzt; wobei der Streifen (S1, S2) geeignet ist, mit der Sicherungseinrichtung (30) zusammenzuwirken, wobei er entweder über oder unter der Erweiterung (32) verläuft, auch wenn Unterwäsche verwendet wird.

6. Schutzkragen (10) gemäß einem der vorherigen Ansprüche, mit zumindest einem Streifen (S1, S2), der dauerhaft an der Sicherungseinrichtung (30) gefügt ist, wobei der Streifen (S1, S2) offen ist und eine Einstell/Schließeinrichtung hat.

7. Schutzkragen (10) gemäß Anspruch 6, wobei die Einstell/Schließeinrichtung einen Riemen zum Festziehen oder Velcro®-Teile hat, die aneinander befestigt werden.

## Revendications

1. Collier (10) pour protéger le cou d'un utilisateur, particulièrement utile pour les motocyclistes, comprenant :
deux demi-colliers (16, 18) qui sont articulés de manière pivotante ensemble au niveau d'une extrémité afin de pouvoir tourner dans un plan sensiblement horizontal, qui peuvent être raccordés ensemble au moins d'un levier de fixation et de déblocage (14) ;
une plaque (42) qui, à l'arrière, s'étend vers le bas à partir du bord inférieur du collier afin d'être centrée par rapport à la colonne vertébrale de l'utilisateur,
un écran protecteur (12) qui s'étend vers le bas, à l'avant,
des moyens de fixation (30) pour raccorder temporairement ou en permanence au moins une sangle (S1, S2) pour fixer le collier (10) sur le corps de l'utilisateur,
**caractérisé en ce que** :
lesdits moyens de fixation comprennent un élément en forme de languette (30) faisant saillie latéralement du collier (10).

2. Collier protecteur (10) selon la revendication 1, **caractérisé en ce que** ledit élément en forme de languette (30) est formé par un corps central (34) qui se termine par une extension en angle droit (32).

3. Collier protecteur (10) selon la revendication 1 ou 2, **caractérisé en ce que** ladite languette (30) a une tête en forme de champignon élargie (32) pour maintenir la sangle (S1, S2) associée en position.

4. Collier protecteur (10) selon l'une quelconque des revendications précédentes, **caractérisé en ce que** lesdits éléments de fixation en forme de languette (32) sont au nombre de deux, un de chaque côté (16, 18) du collier (10).

5. Collier protecteur (10) selon l'une quelconque des revendications précédentes, comprenant en outre au moins une sangle élastique (S1, S2) appropriée pour fixer le collier protecteur (10) sur le corps de l'utilisateur, ladite au moins une sangle élastique (S1, S2) étant agencée afin d'être croisée sur la poitrine ; ladite sangle (S1, S2) étant appropriée pour coopérer avec lesdits moyens de fixation (30) passant sur ou sous l'extension (32), également lorsqu'elle est utilisée sous les vêtements.

6. Collier protecteur (10) selon l'une quelconque des revendications précédentes, comprenant au moins une sangle (S1, S2) assemblée en permanence sur lesdits moyens de fixation (30) ; ladite sangle (S1, S2) étant ouverte et ayant des moyens d'ajustement/fermeture.

7. Collier protecteur (10) selon la revendication 6, dans lequel lesdits moyens d'ajustement/fermeture comprennent une courroie pour le serrage ou des parties de Velcro^{®} à fixer ensemble.
